# EUROPEAN PATENT SPECIFICATION

(11) **EP 4 142 626 B1**
(45) Date of publication and mention of the grant of the patent: **25.06.2025**
(21) Application number: 21725817.7
(22) Date of filing: 27.04.2021
(51) Int. Cl.: A61B 17/42, A61M 25/10, A61M 39/12, A61B 17/12, A61B 17/00

(54) **UTERINE DEVICE FOR TREATING POSTPARTUM HEMORRHAGE**
GEBÄRMUTTERVORRICHTUNG ZUR BEHANDLUNG VON POSTPARTALER BLUTUNG
DISPOSITIF UTÉRIN POUR LE TRAITEMENT D'UNE HÉMORRAGIE POST-PARTUM

(30) Priority: 27.04.2020 IT 202000009013; 27.04.2020 IT 202000009016
(43) Date of publication of application: 08.03.2023
(73) Proprietor: Politecnico di Milano, 20133 Milano (IT)
(72) Inventor: COSTANTINO, Maria Laura, 20133 Milano (IT); DE GAETANO, Francesco, 20133 Milano (IT); OSOULI, Kasra, 20133 Milano (IT); ZANINI, Alberto, 20133 Milano (IT); POSSENTI, Luca, 20133 Milano (IT); GRAZIOSI, Serena, 20133 Milano (IT); CANDIDORI, Sara, 20133 Milano (IT)
(74) Representative: Merli, Silvia
(86) International application number: PCT/IB2021/053454
(87) International publication number: WO 2021/220151

(56) References cited:
- WO-A1-2012/099609
- WO-A1-98/52501
- DE-C1- 19 816 829
- US-A1- 2014 074 142
- US-A1- 2018 360 494
- US-B1- 6 488 317
- US-B1- 8 123 773
- KAMYAR MOLLAZADEH-MOGHADDAM ET AL: "Mechanical Properties of the Every Second Matters for Mothers-Uterine Balloon Tamponade(ESM-UBT) Device: In Vitro Tests", AMERICAN JOURNAL OF PERINATOLOGY REPORTS, vol. 09, no. 04, 1 October 2019 (2019-10-01), pages e376 - e383, XP055759051, ISSN: 2157-6998, DOI: 10.1055/s-0039-1697653

## Description

### Background

Postpartum haemorrhage has an estimated frequency of between 5 and 22% of births and is the leading cause of maternal death in the world. Post partum haemorrhage means a blood loss equal to or greater than 500 cc in the 24 hours following childbirth. Haemorrhage is defined as serious when the blood loss exceeds 1000 cc. There are many causes of postpartum haemorrhage, including: uterine atony (90%), lacerations of the cervix and / or perineum (5%), retention of placental material (4%), coagulation problems, uterine inversion, rupture of uterus.

The administration of uterotonic drugs during the third stage of labor drastically reduces the risk of postpartum haemorrhage. Where drugs are not shown to be sufficient to stop bleeding, the uterine tamponade technique is used. This technique involves compression of the uterine viscus with gauze in order to stretch and tampon the walls of the uterus and thus stop blood loss. However, the technique is undermined by the possibility of the so-called "masking", that is, the loss stops only apparently, the blood continues to flow passing by capillarity through the gauze itself, becoming visible only after some time. Furthermore, it is possible when removing the gauze to forget some of them on the spot.

An alternative to tamponade with gauze involves the use of catheters. These include the insertion of a deflated balloon, through the vagina and cervix, into the uterus and subsequent filling with a saline solution. The balloon, thanks to the pressure exerted on the damaged walls of the uterus, stops the bleeding. One of the most used is the Bakri balloon, a temporary means for achieving haemostasis in cases where conservative management of postpartum uterine bleeding is indicated (Vitthala S et al. Use of Bakri balloon in postpartum haemorrhage: a series of 15 cases. Aust NZJ Obstet Gynaecol. 2009; 49: 191-194). It is a device of a certain complexity which is accompanied by costs that cannot be faced in many disadvantaged geographical areas, which therefore still lack adequate tools to deal with this dangerous emergency.

Rudimentary approaches to remedy the unavailability of adequate equipment have been described and typically use condoms that are inserted into the uterus with the aid of forceps, inflated and then held in place by closing the end with what is available, for example a tie. These approaches remain lacking both from the point of view of sterility and effectiveness, where the closures do not guarantee the necessary tightness to ensure the correct pressure of the balloon on the uterine walls.

As an example, Kamyar Mollazadeh-Moghaddam et al., 2019 in American J Perinatology Reports 9, e376-e383 describe the use of a condom to stop uterine bleeding, connected to a catheter via an O-ring.

WO98 / 52501, US8123773, US2018 / 360494 describe a balloon for uterine insertion, where a balloon is connected to a catheter.

There is therefore a strong need for an effective and low-cost device in the treatment of postpartum haemorrhage.

### Description

The present invention is defined by appended claim 1 and relates to a device for intrauterine insertion capable of providing a haemostatic effect in the uterine cavity.

### Figure description

**Figure 1****:** device for intrauterine insertion.
**Figure 2****:** an embodiment of the seal connector.
**Figure 3****:** an embodiment of the device for intrauterine insertion according to the present invention, with cable gland.
**Figure 4****:** intrauterine insertion device with introducer.
**Figure 5****:** intrauterine introducer.
**Figure 6****:** a further embodiment of the seal connector (A) perspective view with uterine introducer; (B) sectional view.
**Figure 7****:** a further embodiment of a seal connector and uterine introducer.
**Figure** 8: a seal connector, in accordance with the invention, (A, B, C) perspective views.
**Figure 9****:** embodiment of the seal connector of Figure 8, (A, B, C) perspective views without rubber bands.
**Figure 10****:** a further embodiment of a seal connector, with an elastic membrane, perspective view.
**Figure 11****:** an embodiment of the membrane in the seal connector of Figure 10, membrane with an auxetic structure.
**Figure 12****:** a further embodiment of a seal connector, with auxetic structure, (A) front view; (B) longitudinal section.
**Figure 13****:** a further embodiment of a seal connector, (A) perspective view, (B) top view.

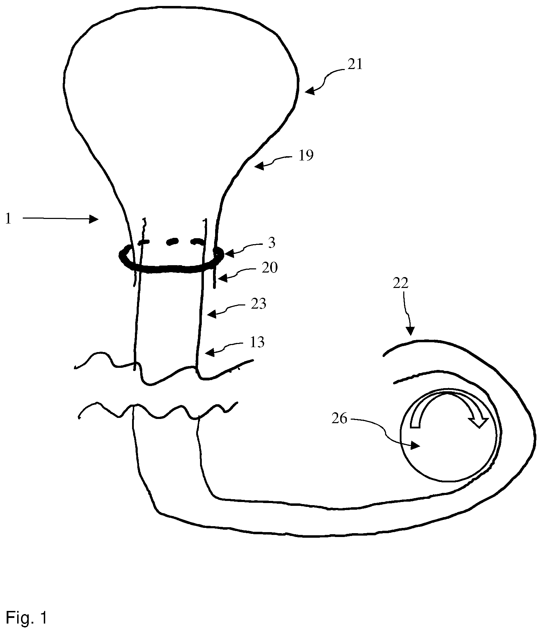

The subject of the present disclosure is a device 1 (Figure 1) for intrauterine insertion which includes:
- an expandable balloon 19, having a proximal end 20 and a distal end 21, sized to fill the uterine cavity during expansion and to exert pressure against its boundary wall on substantially the total surface of said wall;
- a catheter 13 having a proximal end 22 and a distal end 23, where said distal end 23 of said catheter is connected with the proximal end 20 of said balloon 19, and said proximal end 22 of said catheter is connected to a source of pressurized fluid to the balloon through said catheter to effect and maintain its expanded condition,
characterized in that said catheter 13 is connected with a seal connector 3, 15, 60, 70, 80, 100, 120, 130 to said balloon 19.

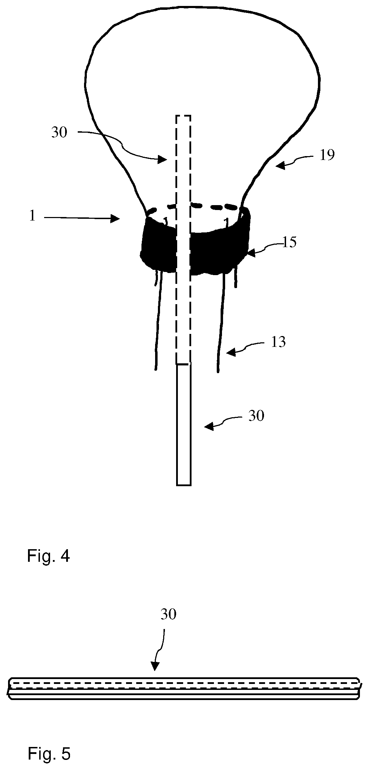

In an embodiment, Figure 4, said device 1 for intrauterine insertion also comprises a uterine introducer 30.

In one embodiment, said uterine introducer 30 is a rigid polygonal section bar with at least 5 sides (Figure 5).

Said uterine introducer 30 has no sharp edges or angles and has a section such as to guarantee the necessary rigidity. In a preferred form, the section of said introducer is cross, star, toothed wheel. Even more preferably, said uterine introducer has a cross section, where the edges are rounded.

The geometry of said uterine introducer 30 is advantageously selected so as to allow the passage of fluid through said catheter 13 into said balloon even during the insertion and positioning of said introducer. In fact, said polygonal section with at least 5 sides ensures that even when said introducer 30 inserted in said catheter 13 is held in the hands, a lumen always remains in said catheter 13 for the passage of fluid.

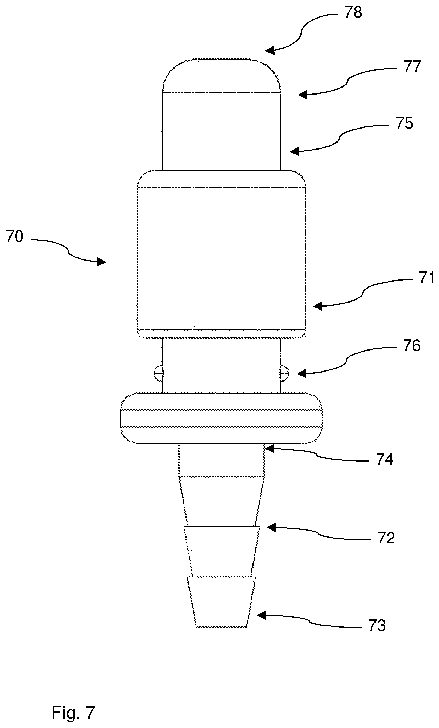

In an alternative form, figure 7, said uterine introducer is a straw 75, where said straw is closed at one end, where said closure leaves no sharp edges. On said closed end, holes are made which allow the passage of fluid. Also this embodiment allows the passage of fluid inside said straw during insertion and positioning. Said closure which leaves no sharp edges avoids the sharp edges that would be present using a rigid straw having both ends open.

In one embodiment, said balloon is a condom.

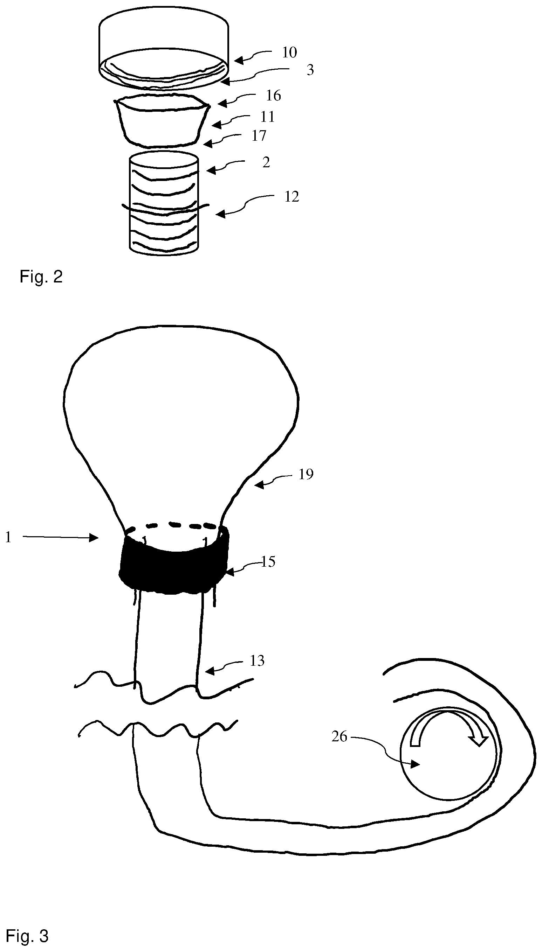

In one embodiment, not forming part of the claimed invention, Figures 2, 3, said seal connector 3 is a cable gland 15. Said cable gland 15 comprises a perforated head 10, a perforated body 12, said head and said body being axially arranged with respect to each other, or along the length of said cable gland, when the cable gland is assembled, with an end portion 2 of said body 12 received in a receiving portion 3 of said head 10; a gasket 11 arranged axially with respect to said head and said body and interposed between them when said cable gland is assembled, where said gasket has an upper portion 16 and a lower portion 17, said lower portion 17 being received in said body and said gasket being tapered downwards, i.e. from said upper portion 16 to said lower portion 17.

Said perforated head and said perforated body engage each other preferably by screwing. Preferably, said perforated head 10 has an internal thread and said perforated body 12 has an external thread, where said internal and external thread are complementary to each other.

In one embodiment, said cable gland is made of polymeric material.

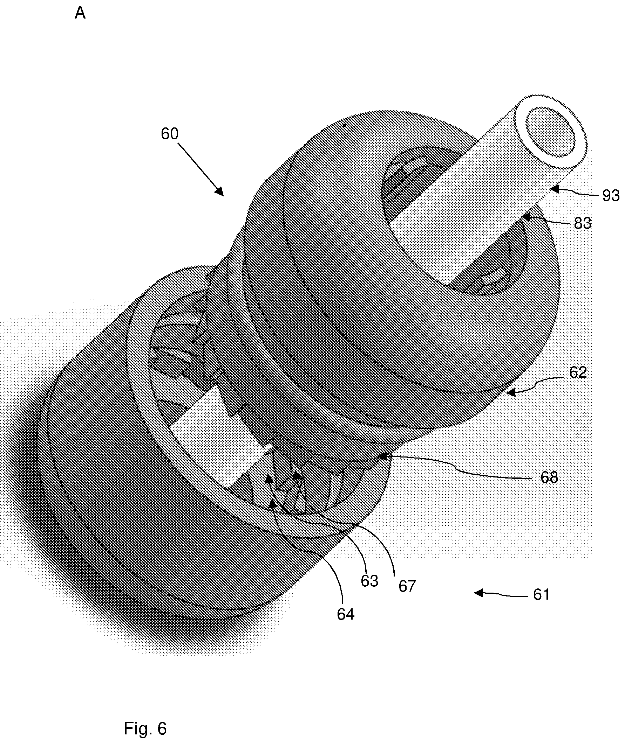

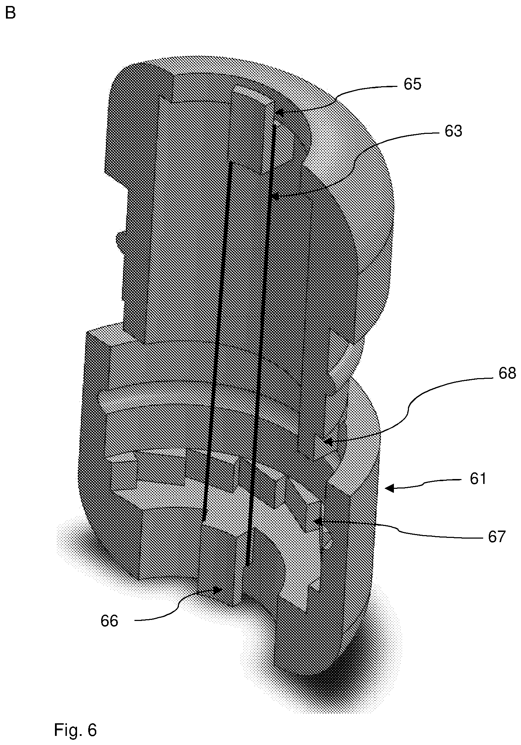

In a further embodiment, not forming part of the claimed invention, said seal connector 3 is of the type shown schematically in Figure 6A, B.

In this embodiment, said seal connector is a seal connector with rubber bands and two rigid gear wheels, seal connector 60, and comprises a first perforated body 61 and a second perforated body 62, mounted axially, i.e. one above the other, in such a way that the hole of said first perforated body 61 is aligned with the hole of said second perforated body 62. Said sealing connector has a length which extends from said first perforated body to said second perforated body. Inside said first and second perforated bodies 61 and 62 are housed axially, or along the length of said seal connector, two rubber bands mounted on special supports. Figure 6B shows a vertical section of said sealing connector 60 which shows the supports 65 and 66 on which the elastic 63 is mounted. In the section that mirrors that of figure 6B, there are two further supports, mirroring those shown in Figure 6B, on which the second elastic is housed.

Said first and said second perforated body 61 and 62 receive, respectively, a first toothed wheel 67 and a second toothed wheel 68. Said first and second toothed wheel 67 and 68 engage each other so as to form a ratchet, that is a mechanical device that allows a rotary movement around the axis in a sense only, where the play linked to the distance between the teeth that allows rotation in the opposite direction is negligible.

The catheter 83 is inserted, crossing it axially, or along its length, into said hermetic connector 60.

Once the balloon has been inserted at the distal end 93 of the catheter 83 and after having inserted the set of catheter plus balloon inside the aforementioned perforated bodies 61 and 62, the same are made to rotate between them, and the rubber bands inside them seal the balloon to the catheter ensuring its seal.

In a further embodiment, not forming part of the claimed invention, said sealing connector 3 is of the type shown schematically in figure 7.

Said sealing connector 70 comprises a perforated head 71 and a hollow tapered body 72.

Said hollow tapered body 72 has a proximal end 73 and a distal end 74. A uterine introducer 75 is grafted onto the distal end 74 of said perforated body 72. Said uterine introducer is a straw, which has a proximal end 76 and a distal end 77. Said uterine introducer 75 is closed to said distal end 77 by means of a closure plug 78, without said closure leaving sharp edges. In said distal end 77 of said uterine introducer 75, there are holes which allow the passage of fluid.

During use, a balloon is inserted on the distal end 77 of said uterine introducer 75 and the set is passed through the perforated head 71 which closes on the introducer 75 through pins 76, so as to ensure the seal.

The device according to this embodiment can always be used but is particularly advantageous in cases of haemorrhage following a caesarean section, where it is necessary to introduce the balloon bound to the sealing connector 70 into the uterus, which is passed through the cervix and then the vagina before being connected via the proximal end 73 to the catheter 13.

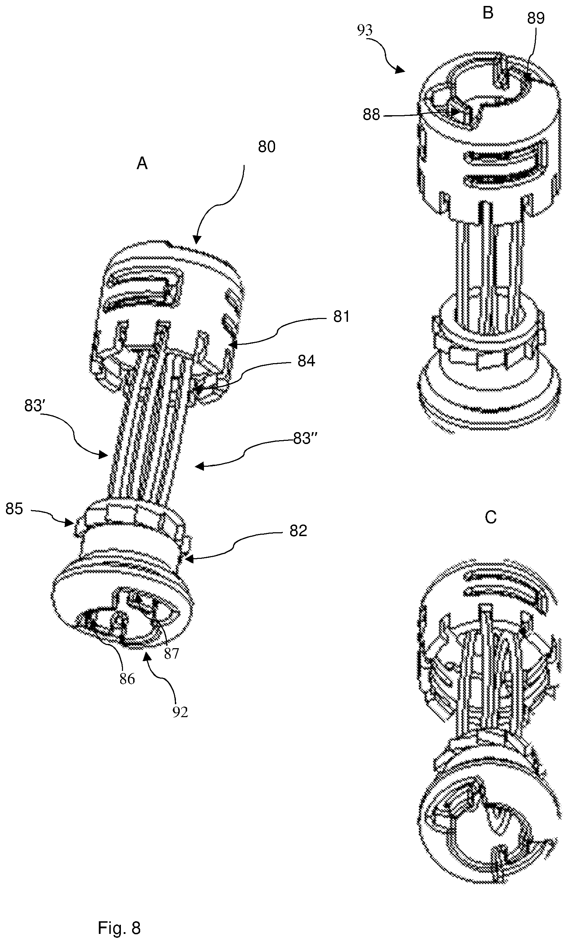

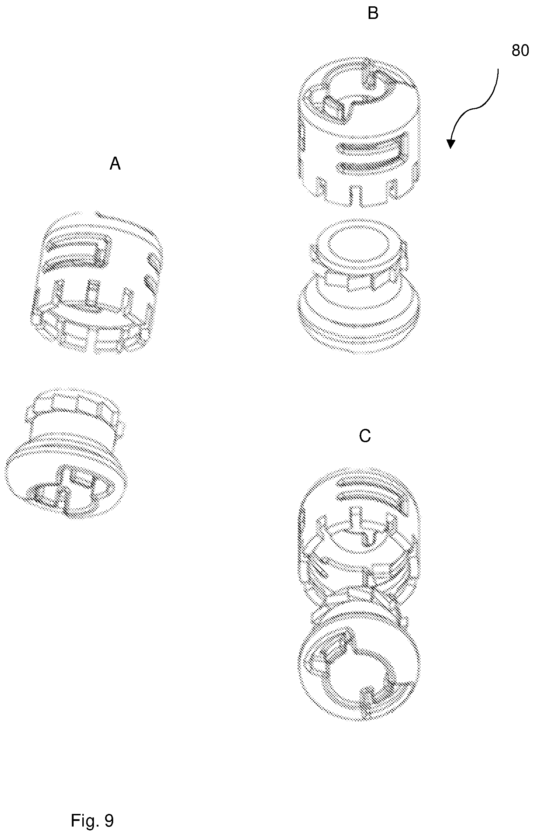

According to the claimed invention, said sealing connector is of the type schematized in figure 8 and figure 9.

Said seal connector in this embodiment is a seal connector with rubber bands and two toothed wheels, one of which is radially deformable, seal connector 80, and comprises a first perforated body 81 and a second perforated body 82, mounted axially. Two elastic bands, 83' and 83", are axially housed inside said first and second perforated bodies 81 and 82. In figure 8A, the bottom perspective view of said sealing connector 80 shows the lower base 92 of said second perforated body 82 and the supports 86 and 87 on which said first and said second elastic 83' and 83" are conveniently mounted, respectively . The view of figure 1B shows the upper base 93 of said first perforated body 81 and the supports 88 and 89, mirroring those present on said first body and shown in figure 8A, on which said first and said second elastic 83' and 83" are conveniently mounted. Said first elastic 83' is housed on said supports 86 and 88, said second elastic 83"' is housed on said supports 87 and 89.

The distinctive feature of said connector is to be found in the fact that said first and second perforated bodies 81 and 82 accommodate, respectively, a first toothed wheel 84 capable of deforming radially and a second rigid toothed wheel 85. Said first and second toothed wheel 84 and 85 engage with each other so as to form a ratchet, that is a mechanical device that allows a rotary movement around the axis in one direction only, where the play linked to the distance between the teeth that allows rotation in the opposite direction is negligible.

Figure 9 shows the same perspective views 9A, 9B, 9C, in the absence of the rubber bands.

The catheter is inserted, crossing it axially, into said seal connector 80, as described for the embodiment schematized in Figure 6.

Once the balloon has been inserted at the distal end of the catheter and after having inserted the set of catheter plus balloon inside the aforementioned perforated bodies 81 and 82, assembled with said first and said second elastic as described, they are made to rotate between them, and said rubber bands seal the balloon to the catheter ensuring its tightness.

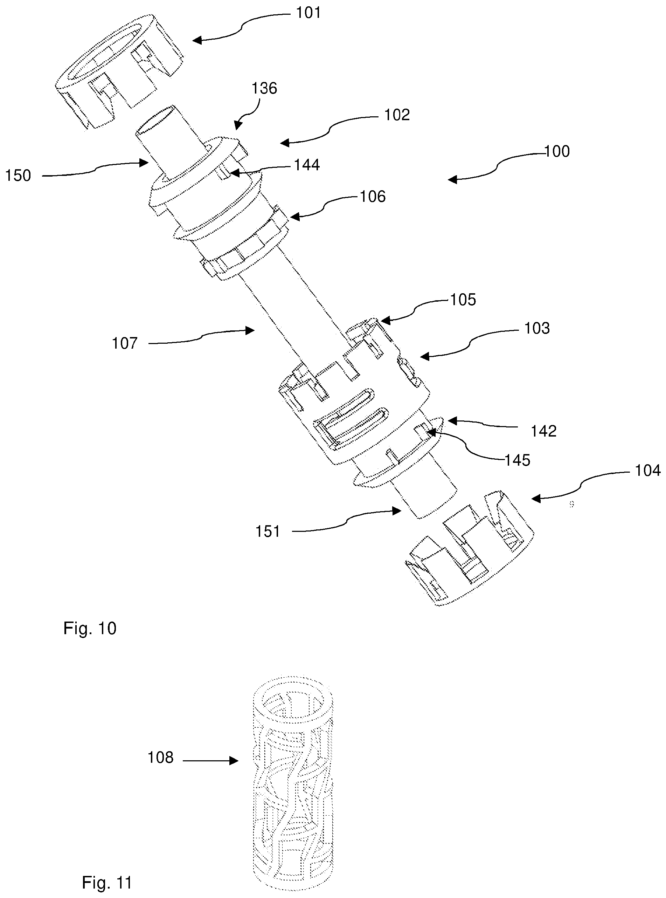

In a further embodiment, not forming part of he claimed invention, said sealing connector is of the type schematized in Figure 10 and comprises a membrane, or cylinder, elastic or auxetic.

Said seal connector with membrane or cylinder elastic or auxetic, seal connector 100, comprise four perforated bodies 101, 102, 103, 104 each having an upper end and a lower end. Once axially assembled, said four perforated bodies are arranged in such a way that said perforated bodies 102 and 103 are located in the center, said perforated bodies 101 and 104 are located, respectively, at the distal end and at the proximal end of said seal connector 100. Said perforated body 103 comprises a first rigid toothed wheel 105. Said perforated body 102 comprises a second toothed wheel 106, capable of deforming radially. An elastic cylinder 107 is inserted inside said perforated bodies, which has an upper portion 150 and a lower portion 151. Said upper portion 150 is folded over the upper end of the perforated body 102. In said upper end of the perforated body 102 there is a mechanism 136 which allows its locking when the perforated body 101 is axially mounted on said perforated body 102.

Similarly, said lower portion 151 is folded onto the lower end of said perforated body 103, an end in which there is a mechanism 142 that allows locking it when the perforated body 104 is axially mounted on said perforated body 103.

Constrained said elastic cylinder 107 to said upper end of the perforated body 102 and to the lower end of said perforated body 103, said perforated body 102 is assembled to said perforated body 103 and locked.

Once assembled, the toothed wheels 105 and 106 engage with each other so as to form a ratchet, that is a mechanical device that allows a rotary movement around the axis in one direction only, where the play linked to the distance between the teeth that allows rotation in the opposite sense it is negligible.

Said perforated bodies 102 and 103 also comprise teeth 144 and 145, respectively. Said teeth 144 and 145 have the purpose of not allowing the rotation of said perforated bodies 102 and 103 when they are put into rotation in implementing the ratchet mechanism.

The catheter is inserted, crossing it axially, into said seal connector 100, as described in relation to the embodiment of Figure 6.

Once the balloon has been inserted at the distal end of the catheter and after having inserted the set of catheter plus balloon inside the aforementioned perforated bodies 101, 102, 103 and 104, the perforated bodies 102 and 103, to which the perforated bodies 101 and 104 are rigidly connected, respectively, are made to rotate with each other, and the membrane inside them seals the balloon to the catheter, thus guaranteeing its tightness.

In an alternative embodiment, not forming part of the claimed invention, the elastic membrane 107 is replaced by an auxetic geometry of deformable material 108, as schematized in figure 11.

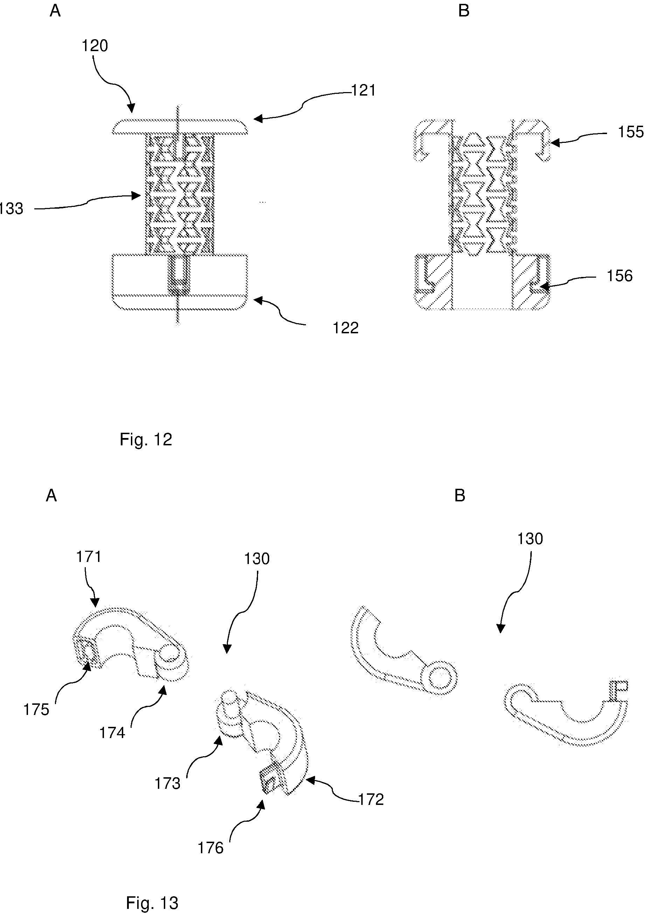

In a further embodiment, not forming part of the claimed invention, said seal connector is characterized by a hook mechanism and comprises an auxetic membrane, seal connector 120. Said seal connector 120, shown schematically in Figure 12, comprises a first perforated body 121 and a second perforated body 122, axially mounted. The two axial ends of an auxetic cylinder 133 are constrained inside said first and second perforated bodies. The perforated body 121 has a hook mechanism 155 which fits into the seat 156 of the perforated body 122, blocking any axial movement.

The catheter is inserted, crossing it axially, into said seal connector 120, as described in relation to the embodiment of Figure 6.

Once the balloon has been inserted at the distal end of the catheter and after having inserted the set of catheter plus balloon inside the aforementioned perforated bodies 121 and 122, the perforated bodies are axially approached to facilitate their interlocking by means of components 155 and 156 , and this axial compression causes a reduction in the diameter of the auxetic membrane inside them, sealing the balloon to the catheter ensuring its tightness.

In a further embodiment, not forming part of the claimed invention, said sealing connector is shown in figure 13, seal connector 130, and it is composed of two bodies 171 and 172, hinged together by means of cylindrical pin 173 which, together with bearing 174, allows said connector a rotational motion around the axis of the cylinder itself.

Once the balloon has been inserted at the distal end of the catheter and after having inserted the catheter and balloon assembly, the two bodies 171 and 172, after being hinged, close by means of a clip 176 on the body 172 which fits into a cavity 175 on body 171, sealing the balloon to the catheter ensuring its tightness.

Advantageously, the device according to the present invention is characterized by a great simplicity of assembly, where the elements that compose it are either easy to manufacture or even already available, not to the detriment of its effectiveness, whereas the seal connector 3, 15 , 60, 70, 80, 100, 120, 130 allows to connect the end of the catheter 13 to the balloon 19 in a sealed way, so as to avoid any possible leakage in the passage of fluid through the tube to inflate the balloon, ensuring the due efficiency of tamponade.

Advantageously, said uterine introducer 30, inserted in said catheter 13, makes it less flexible and more rigid in the final part, thus assisting the operator in inserting the same and positioning the balloon.

The solution according to the present invention therefore solves the problem of having a simple and therefore economical device but with efficiency characteristics comparable to the characteristics of the more complex and therefore expensive Bakri devices.

## Claims

1. A device (1) for intrauterine insertion which comprises:
- an expandable balloon (19) having a proximal end (20) and a distal end (21), sized to fill the uterine cavity during expansion and to exert pressure against its boundary wall on substantially the total surface of said wall;
- a catheter (13) having a proximal end (22) and a distal end (23), where said distal end (23) of said catheter is connected with the proximal end (20) of said balloon (19), and said proximal end (22) of said catheter is connected to a source of fluid and means (26) for supplying pressurized fluid to the balloon (19) through said catheter to effect and maintain its expanded condition,
wherein said catheter (13) is connected with a seal connector (80) to said balloon (19), **characterized in that** said seal connector (80) comprises:
- a first perforated body (81) and a second perforated body (82), mounted axially, i.e. along the axis;
- two elastic bands, (83') and (83") axially housed inside said first and second perforated bodies (81) and (82), where a first elastic (83') is mounted on supports (86) and (88) comprised, respectively, on said second and on said first perforated body and a second elastic (83") is mounted on supports (87) and (89) present, respectively, on said second and on said first perforated body;
where said first and said second perforated body receive, respectively, a first toothed wheel (84) capable of radially deforming and a second rigid toothed wheel (85), where said first and second toothed wheel (84) and (85) engage between them so as to form a ratchet, that is a mechanical device that allows a rotary movement around the axis in one direction only.

2. The device according to claim 1, which also comprises a uterine introducer (30).

3. The device according to claim 2, wherein said uterine introducer (30) is a rigid rod with a polygonal section with at least 5 sides and has no sharp edges or corners.

4. The device according to claim 2, where said uterine introducer (30) is a straw, where said straw is closed at one end without said closure leaving sharp edges and said closure comprise holes that allow the passage of fluid.

5. The device according to one of claims 1-4, wherein said seal connector (3) is made of polymeric material.

6. A device according to one of claims 1 to 5 for use in tamponade of postpartum intrauterine haemorrhages.

## Patentansprüche

1. Vorrichtung (1) zum intrauterinen Einsetzen, die Folgendes umfasst:
- einen ausdehnbaren Ballon (19), der ein proximales Ende (20) und ein distales Ende (21) aufweist, mit einer Größe, um die uterine Höhle während der Ausdehnung zu füllen und Druck gegen deren Begrenzungswand auf die im Wesentlichen gesamte Oberfläche der Wand auszuüben;
- einen Katheter (13), der ein proximales Ende (22) und ein distales Ende (23) aufweist, wobei das distale Ende (23) des Katheters mit dem proximalen Ende (20) des Ballons (19) verbunden ist, und das proximale Ende (22) des Katheters mit einer Fluidquelle und einer Einrichtung (26) zum Zuführen von unter Druck stehendem Fluid zu dem Ballon (19) durch den Katheter verbunden ist, um dessen ausgedehnten Zustand zu bewirken und aufrechtzuhalten,
wobei der Katheter (13) mit einem Dichtungsverbinder (80) mit dem Ballon (19) verbunden ist, **gekennzeichnet dadurch, dass** der Dichtungsverbinder (80) Folgendes umfasst:
- einen ersten perforierten Körper (81) und einen zweiten perforierten Körper (82), die axial, d. h. entlang der Achse, befestigt sind;
- zwei elastische Bänder, (83') und (83"), die axial im Inneren des ersten und des zweiten perforierten Körpers (81) und (82) untergebracht sind, wobei ein erstes elastisches Band (83') auf Trägern (86) und (88) befestigt ist, die jeweils auf dem zweiten und auf dem ersten perforierten Körper umfasst sind, und ein zweites elastisches Band (83") auf Trägern (87) und (89) befestigt ist, die jeweils auf dem zweiten und auf dem ersten perforierten Körper vorhanden sind;
wobei der erste und der zweite perforierte Körper jeweils ein erstes Zahnrad (84), das in der Lage ist, sich radial zu verformen, und ein zweites starres Zahnrad (85) aufnehmen, wobei das erste und das zweite Zahnrad (84) und (85) so im Eingriff miteinander sind, dass sie eine Ratsche ausbilden, welche eine mechanische Vorrichtung ist, die eine Drehbewegung um die Achse in nur eine Richtung erlaubt.

2. Vorrichtung nach Anspruch 1, die auch eine uterine Einführeinrichtung (30) umfasst.

3. Vorrichtung nach Anspruch 2, wobei die uterine Einführeinrichtung (30) ein starrer Stab mit einem polygonalen Querschnitt mit mindestens 5 Seiten ist und keine scharfen Kanten oder Ecken aufweist.

4. Vorrichtung nach Anspruch 2, wobei die uterine Einführeinrichtung (30) ein Strohhalm ist, wobei der Strohhalm an einem Ende geschlossen ist, ohne dass der Verschluss scharfe Kanten zurücklässt, und der Verschluss Löcher umfasst, die den Durchtritt von Fluid erlauben.

5. Vorrichtung nach einem der Ansprüche 1 bis 4, wobei der Dichtungsverbinder (3) aus Polymermaterial hergestellt ist.

6. Vorrichtung nach einem der Ansprüche 1 bis 5 zur Verwendung in einer Tamponade von intrauterinen Blutungen nach der Geburt.

## Revendications

1. Dispositif (1) pour insertion intra-utérine qui comprend :
- un ballon extensible (19) ayant une extrémité proximale (20) et une extrémité distale (21), dimensionné pour remplir la cavité utérine pendant l'expansion et pour exercer une pression contre sa paroi limitrophe sur sensiblement la totalité de la surface de ladite paroi ;
- un cathéter (13) ayant une extrémité proximale (22) et une extrémité distale (23), dans lequel ladite extrémité distale (23) dudit cathéter est connectée à l'extrémité proximale (20) dudit ballon (19), et ladite extrémité proximale (22) dudit cathéter est connectée à une source de fluide et à des moyens (26) pour fournir un fluide sous pression au ballon (19) à travers ledit cathéter afin d'effectuer et de maintenir son état expansé,
dans lequel ledit cathéter (13) est connecté avec un connecteur d'étanchéité (80) audit ballon (19), **caractérisé en ce que** ledit connecteur d'étanchéité (80) comprend :
- un premier corps perforé (81) et un deuxième corps perforé (82), montés axialement, c'est-à-dire le long de l'axe ;
- deux bandes élastiques (83') et (83") logées axialement à l'intérieur desdits premier et deuxième corps perforés (81) et (82), dans lequel un premier élastique (83') est monté sur des supports (86) et (88) compris, respectivement, sur ledit deuxième et sur ledit premier corps perforés et un deuxième élastique (83") est monté sur des supports (87) et (89) présents, respectivement, sur ledit deuxième et sur ledit premier corps perforés ;
dans lequel ledit premier et ledit deuxième corps perforés reçoivent, respectivement, une première roue dentée (84) capable de se déformer radialement et une deuxième roue dentée (85) rigide, dans lequel ladite première et ladite deuxième roues dentées (84) et (85) s'engagent entre elles de manière à former un cliquet, c'est-à-dire un dispositif mécanique qui permet un mouvement rotatif autour de l'axe dans une seule direction.

2. Dispositif selon la revendication 1, qui comprend également un introducteur utérin (30).

3. Dispositif selon la revendication 2, dans lequel ledit introducteur utérin (30) est une tige rigide avec une section polygonale d'au moins 5 côtés et qui ne présente pas d'arêtes ou d'angles vifs.

4. Dispositif selon la revendication 2, dans lequel ledit introducteur utérin (30) est une gaine, dans lequel ladite gaine est fermée à une extrémité sans que ladite fermeture ne laisse d'arêtes vives et ladite fermeture comprend des trous qui permettent le passage d'un fluide.

5. Dispositif selon l'une des revendications 1 à 4, dans lequel ledit connecteur d'étanchéité (3) est fait d'un matériau polymère.

6. Dispositif selon l'une des revendications 1 à 5 pour une utilisation dans la tamponnade d'hémorragies intra-utérines post-partum.
